(19) **Europäisches Patentamt / European Patent Office / Office européen des brevets**

(11) **EP 2 344 034 B1**

(12) **EUROPEAN PATENT SPECIFICATION**

(45) Date of publication and mention
of the grant of the patent:
**30.09.2015 Bulletin 2015/40**

(21) Application number: **09745115.7**

(22) Date of filing: **07.10.2009**

(51) Int Cl.:
*A61B 5/02* (2006.01)     *A61B 5/026* (2006.01)
*A61B 5/053* (2006.01)    *G08B 21/04* (2006.01)

(86) International application number:
**PCT/IB2009/054392**

(87) International publication number:
**WO 2010/041205 (15.04.2010 Gazette 2010/15)**

(54) **MONITORING OF ACUTE STROKE PATIENTS**

ÜBERWACHUNG VON PATIENTEN MIT AKUTEM SCHLAGANFALL

SURVEILLANCE DE PATIENTS PRÉSENTANT UN ACCIDENT VASCULAIRE CÉRÉBRAL

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.10.2008 US 103287 P**

(43) Date of publication of application:
**20.07.2011 Bulletin 2011/29**

(73) Proprietor: **Orsan Medical Technologies Ltd.**
**42504 Natania (IL)**

(72) Inventors:
• **POUPKO, Ben Zion**
  **74051 Nes Ziona (IL)**
• **RAPPAPORT, Alon**
  **69407 Tel-Aviv (IL)**
• **BEN-ARI, Shlomi**
  **30550 Binyamina (IL)**

(74) Representative: **Dennemeyer & Associates S.A.**
**55, rue des Bruyères**
**1274 Howald (LU)**

(56) References cited:
WO-A1-2006/011128     WO-A1-2006/134501
DE-A1- 10 061 189       RU-C1- 2 141 249
US-A- 5 265 615         US-A1- 2007 287 899
US-A1- 2008 004 904     US-A1- 2008 200 787

• LOVETT DOUST J W ET AL: "Aspects of the cerebral circulation during non REM sleep in healthy controls and psychiatric patients, as shown by rheoencephalography" PSYCHOPHYSIOLOGY 1975, vol. 12, no. 5, 1975, pages 493-498, XP002572590 ISSN: 0048-5772

## Description

This invention relates to a system and method for monitoring an acute stroke patient

FIELD AND BACKGROUND OF THE INVENTION

The present invention, in some embodiments thereof, relates to a method of monitoring acute stroke patients using impedance plethysmography (IPG) and/or photoplethysmography (PPG) and, more particularly, but not exclusively, to monitoring ischemic stroke patients and sub-arachnoid hemorrhage (SAH) patients for significant changes in cerebral hemodynamic parameters.

A number of cerebral hemodynamic parameters may be clinically useful for diagnosing strokes, trauma, and other conditions that can affect the functioning of the cerebrovascular system. These parameters include regional cerebral blood volume, cerebral blood flow, cerebral perfusion pressure, mean transit time, time to peak, and intracranial pressure. Many methods that are used to measure these parameters, while giving accurate results, are not practical to use for continuous monitoring, or for initial diagnosis outside a hospital setting, because they are invasive, or because they require expensive and/or non-portable equipment. Such methods include inserting a probe into the cerebrospinal fluid or into an artery, computed tomography (CT) perfusion computed tomography (PCT), positron emission tomography (PET), magnetic resonance imaging (MRI), and transcranial Doppler ultrasound (TCD). Some of this prior art is reviewed in US patent application 11/610,553, published as US2007/0287899 and WO2008/072223, and in the other related applications listed above.

The use of perfusion computed tomography for finding cerebral hemodynamic parameters, and the use of these parameters in evaluating and choosing courses of treatment for stroke patients, is described by Christian Baumgartner et al, "Functional Cluster Analysis of CT Perfusion Maps: A New Tool for Diagnosis of Acute Strokes," J. of Digital Imaging 18, 219-226 (2005); by Roland Bruening, Axel Kuettner and Thomas Flohr, Protocols for Multislice CT (Springer, 2005), especially on page 96; by Ellen G. Hoeffner et al, "Cerebral Perfusion CT: Technique and Clinical Applications," Radiology 231, 632-644 (2004); and by Hiroshi Hagiwara et al, "Predicting the Fate of Acute Ischemic Lesions Using Perfusion Computed Tomography, "J. Comput. Assist. Tomogr. 32, 645-650 (2008).

A. M. Weindling, N. Murdoch, and P. Rolfe, "Effect of electrode size on the contributions of intracranial and extracranial blood flow to the cerebral electrical impedance plethysmogram," Med. & Biol. Eng. & Comput. 20, 545-549 (1982) describes measurements of blood flow in the head, using separate current and voltage electrodes on the front and back of the head, and measuring the peak-to-peak change in impedance over a cardiac cycle to find the blood flow. A tourniquet was placed around the head to temporarily stop the scalp blood flow, and then released, in order to determine how much of the measured blood flow was due to scalp blood flow, and how much was due to intracranial blood flow. The scalp blood flow was considered to be completely cut off when there was no detectable variation in the signal from a PPG sensor at the cardiac frequency.

J. Gronlund, J. Jalonen, and I. Valimaki, "Transcephalic electrical impedance provides a means for quantifying pulsatile cerebral blood volume changes following head-up tilt," Early Human Development 47 (1997) 11-18, describe electrical impedance measurements of the head in premature newborn infants. Changes in impedance associated with the cardiac cycle are said to reflect changes in total cerebral blood volume, and earlier papers are referenced which are said to demonstrate this. Variability in impedance, in the range of 1.5 to 4 Hz, was found to decrease by 27%, on average, when the infants' heads were tilted up by 20 degrees. An earlier paper describing related research by the same group is J. Gronlund et al, "High Frequency Variability of Transcephalic Electrical Impedance: A New Parameter for Monitoring of Neonatal Cerebral Circulation?", Proceedings of the Annual International Conference of the Engineering in Medicine and Biology Society, Paris, October 29-November 1, 1992, New York, IEEE, US, Vol. 6 Conf. 14, 29 October 1992, pages 2513-2515.

Rheoencephalography (REG) is a technique that uses bio-impendance measurements of the head to obtain information on about cerebral blood circulation and circulatory problems. Generally, changes in impedance Z across the head, for a particular arrangement of electrodes, are measured as a function of time t over a cardiac cycle, and sometimes over a breathing cycle, due to changes in the volume and distribution of blood in the head. As described by W. Traczewski et al, "The Role of Computerized Rheoencephalography in the Assessment of Normal Pressure Hydrocephalus," J. Neurotrauma 22, 836-843 (2005), REG is commonly used to measure or diagnose problems with circulatory resistance, and problems with arterial elasticity. In patients with normal pressure hydrocephalus, for example, Traczewski et al find two different patterns in Z(t), depending on the elasticity of the small cerebral arteries. The pattern of Z(t) seen in a given patient is said to be useful for making predictions about the likely outcome of different treatments for the hydrocephalus. These patients all had similar, normal values of ICP.

G. Bonmassar and S. Iwaki, "The Shape of Electrical Impedance Spectrosopy (EIS) is altered in Stroke Patients," Proceedings of the 26th Annual Conference of IEEE EMBS, San Francisco, CA, USA, September 1-5, 2004, describes a system that uses electrical impedance to measure an asymmetry in the distribution of cerebral spinal fluid that is present in stroke patients, but not in healthy volunteers. The system uses 10 electrodes placed symmetrically around the subject's head, and passes white noise current at 0 to 25 kHz between any selected pair of electrodes, while measuring the potentials at all the electrodes. The system was found to work best if current was passed between the front and back of the head, but the paper also describes passing current between symmetrically placed electrodes on the left and right sides of the head.

WO 02/071923 to Bridger et al describes measuring and analyzing pulse waveforms in the head obtained from acoustic signals. Head trauma patients, and to a lesser extent stroke patients, are found to have differences from normal subjects. Trauma and stroke patients are found to have higher amplitudes at harmonics of the heart rate, at 5 to 10 Hz, than normal subjects do.

Yu. E. Moskalenko et al, "Slow Rhythmic Oscillations within the Human Cranium: Phenomenology, Origin, and Informational Significance," Human Physiology 27, 171-178 (2001), describes the use of electrical impedance measurements of the head, and TCD ultrasound measurements, to study slow waves, at frequencies of 0.08 to 0.2 Hz, that are apparently related to regulation of blood supply and oxygen consumption in the brain, and the circulation of cerebrospinal fluid. The studies were done with healthy subjects and with patients suffering from intracranial hypertension. A. Ragauskas et al, "Implementation of non-invasive brain physiological monitoring concepts," Medical Engineering and Physics 25, 667-687 (2003), describe the use of ultrasound to non-invasively monitor such slow waves, as well as pulse waves at the cardiac frequency, in intracranial blood volume, in head injury patients, and find that they can be used to determine intracranial pressure.

Additional background art includes WO 02/087410 to Naisberg et al; Kidwell CS et al, Comparison of MRI and CT for detection of acute intracerebral hemorrhage. JAMA; 2004: 292: 1823-1830; Horowitz SH et al, Computed tomographic-angiographic findings within the first 5 hours of cerebral infarction, Stroke; 1991: 22 1245-1253; The ATLANTIS, ECASS, and NINDS rt-PA study group investigators, Association of outcome with early stroke treatment: Pooled analysis of ATLANTIS, ECASS, and NINDS rt-PA stroke trials, Lancet; 363: 768-774; Albers G et al, Antithrombotic and thrombolytic therapy for ischemic stroke: The seventh ACCP conference on antithrombotic and thrombolytic therapy, Chest 2004; 126: 483-512; Kohrmann M et a,. MRI versus CT-based thrombolysis treatment within and beyond the 3 hour time window after stroke onset: a cohort study, Lancet Neurol 2006; 5:661-667; Albers GW et al, Magnetic resonance imaging profiles predict clinical response to early reperfusion: The diffusion and perfusion imaging evaluation for understanding stroke evolution (DEFUSE) study, Ann Neurol 2006: 60: 508-517; Johnston SC et al, National stroke association guidelines for the management of transient ischemic attacks, Ann Neurol 2006; 60: 301-313.

Examples of known systems and methods for monitoring an acute stroke patient are disclosed in WO 2006/011128. and WO2006/134501,

In US 2008/200787 A1, there is disclosed a system for monitoring an acute stroke patient according to the preamble of claim 1. US 2008/200787 A1 describes a system and method of estimating blood flow in a brain, comprising causing currents to flow inside the head by producing electric fields inside the head, measuring at least changes in the electric fields and the currents, and estimating changes in the blood volume of the head, using the measurements of the electric fields and the currents, where the current are produced in children or using electrodes at or near holes in the skull.

US2008/004904 describes a medical system including a network, one or more medical data collection appliances coupled to the network, each appliance transmitting data conforming to an interoperable format, and a server coupled to the network to store data for each individual in accordance with the interoperable format.

RU 2141249 describes a method for diagnosing and predicting hypertension disease in patients before thirty.

LOVETT DOUST J W; ET AL "Aspects of the cerebral circulation during non REM sleep in healthy controls and psychiatric patients, as shown by rheoencephalography " PSYCHOPHYSIOLOGY, 1975 Vol:12, Nr:5, Page(s):493 - 498, XP002572590 , US - ISSN 0048-5772, describes another example of known method of monitoring an acute stroke patient.

US2007/287899 describes a method of estimating at least one intracranial hemodynamic parameter in a subject, the method comprising: obtaining data of changes in electrical impedance across the subject's head as a function of time, analyzing the data, and estimating one or more of intracranial pressure, cerebral blood volume, and a factor related to at least one of cerebral perfusion pressure and a mean transit time through cerebral capillaries,

DIE 100 61 189 A1 describes and system and method for continuous determination of the arterial blood pressure uses the pulse transition time (PTT) in which the time of pulse arrival at two different body points is measured and then the difference between the two determined, from which a value for blood pressure is determined, each sensor having two electrodes that measure the impedance of the body region and the mean arterial blood pressure from the corresponding PTT.

It is against the background, and the limitations and problems associated therewith, that the present invention has been developed.

To achieve this, the system for monitoring an acute stroke patient of the invention is characterized by the features claimed in the characterizing part of claim 1, and the invention provides a method of monitoring an acute stroke patient according to claim 26.

## SUMMARY OF THE INVENTION

An aspect of some embodiments of the invention concerns a method of continuously or frequently monitoring acute stroke patients, using IPG and/or PPG, to estimate cerebral hemodynamic parameters, and to detect changes in these parameters that might require immediate medical intervention.

There is thus provided, in accordance with an exemplary embodiment of the invention, a system as defined in claim 1, and a method as defined in claim 26. Further advantageous features are defined in dependent claims 2-25.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 schematically shows a cerebral perfusion monitoring system, monitoring an acute stroke patient in a hospital, according to an exemplary embodiment of the invention ;
FIG. 2 is a flowchart showing a method of monitoring a patient using the system in FIG. 1;
FIG. 3 is a more detailed view schematically showing an exemplary IPG electrode structure and PPG sensor that can be used in the system in FIG. 1, placed on the head of a patient;
FIG. 4 is a more detailed schematic view of the IPG electrode structure shown in FIG. 3;
FIG. 5 is a more detailed schematic view of the PPG sensor shown in FIG. 3; and
FIG. 6A schematically shows IPG and IPG signals for a patient with high global CBV, and FIG. 6B schematically shows IPG and PPG signals for a patient with low global CBV, illustrating a method of analyzing IPG and PPG signals according to an exemplary embodiment of the invention.

DESCRIPTION OF EMBODIMENT OF THE INTENTION

The present invention, in some embodiments thereof, relates to a method of monitoring acute stroke patients using impedance plethysmography (IPG) and/or photoplethysmography (IPG) and, more particularly, but not exclusively, to monitoring ischemic stroke patients and sub-arachnoid hemorrhage (SAH) patients for significant changes in cerebral hemodynamic parameters.

An aspect of some embodiments of the invention concerns a method of monitoring an acute stroke patient using impedance plethysmography (IPG) and/or photoplethysmography (PPG), to detect significant changes in cerebral hemodynamic parameters that might require medical intervention, and to alert medical personnel when such changes are detected. The patient is monitored continuously, or at least at frequent intervals, for example data is obtained at least once an hour, or at least twice an hour, or at least once every 10 minutes, or every 5 minutes, or every minute. Monitoring at least once an hour may allow medical intervention to be performed successfully, even with a few unsuccessful attempts, within the typical time window of 3 hours before penumbral brain tissue is permanently damaged, and more frequent monitoring is inexpensive and improves the safety margin. The duration of monitoring is at least six hours, or at least 12 hours, or at least 24 hours, or at least 48 hours. Monitoring for these durations covers the periods when patients are most likely to develop complications, following an initial stroke, or following thrombolytic therapy or endovascular procedures for treating a stroke. The longer the time, the less likely complications are to develop.

Continuous monitoring means that once enough IPG and/or PPG data has been accumulated to analyze in order to estimate the cerebral hemodynamic parameters, for example several cardiac cycles worth of data, more data begins to be accumulated without interruption, in order to make the next estimate of the cerebral hemodynamic parameters.

In an exemplary embodiment of the invention, continuous or frequent monitoring is made practical by the non-invasive nature, lack of ionizing radiation, relatively small size, and/or relatively low cost of the equipment for IPG and PPG measurements, in contrast to prior art methods of measuring cerebral hemodynamic parameters, such as perfusion CT and perfusion MRI, which are not suitable to use for continuous or very frequent monitoring. The timely medical intervention, for example within an hour or less, made possible by such continuous or frequent monitoring can be critical to preventing or minimizing brain damage in the patient. For example, the patient may be an ischemic stroke patient, and a change in cerebral hemodynamic parameters may indicate a hemorrhagic transformation on the ischemia, which is a common complication especially if the patient has received thrombolytic therapy. Other changes in hemodynamic parameters may indicate a new ischemia, or edema, or high blood pressure which can increase the risk of cerebral hemorrhage. Alternatively, the patient is a sub-archnoid hemorrhage (SAH) patient, and a change in cerebral hemodynamic parameters may indicate vasospasm, which is a major cause of mortality and morbidity in SAH patients.

Optionally, values of one or more standard cerebral hemodynamic parameters in clinical use, such as cerebral blood flow (CBF), cerebral blood volume (CBV), mean transit (MTT), and time to peak (TTP), are estimated from IPG and/or PPG signals, and medical personnel are alerted if one or a combination of these standard parameters changes, or fails to change when it was expected to, in a way that indicates medical intervention is needed. Alternatively or additionally, a condition for alerting medical personnel is formulated directly in terms of characteristics of the IPG and/or PPG signals. In either case, this is referred to herein as alerting medical personnel in response to changes in one or more measures of cerebral hemodynamics.

A variety of methods of analyzing IPG and PPG signals may optionally be used to obtain estimate standard parameters or to obtain other measures of cerebral hemodynamics. Typically the measures depend on the behavior of the signal as a function of phase of the cardiac cycle, although measures based on behavior over longer time scales, such as a slow wave amplitude, may also be found. A correlation between slow wave amplitude and volume of stroke lesion is described, for example, in related provisional application 61/103,287, and in the co-filed application titled "Measurement of Cerebral Hemodynamic Parameters." The signals may be smoothed, or averaged over multiple cardiac cycles, or transformed in other ways, and noisy or outlying cardiac cycles may be excluded. The measures may pertain to an effective rise time interval of the signal during a cardiac cycle, defined in various ways, or to an effective fall time interval. The measures may depend on integrals of the signal, for example integrals over an effective rise time or over the whole cardiac cycle, and may depend on weighted integrals, in which, for example, the signal is weighed with a function that falls off smoothly at the limits of the integral, before performing the integration. Obtaining the measures may involve comparing measures found from substantially the same algorithm applied to different signals, for example comparing IPG and PPG signals, or comparing signals based on data pertaining to different sides of the head, or comparing a signal from data pertaining symmetrically to both sides of the head to a signal from data pertaining to only one side of the head. The measures may be dimensionless, not depending on an amplification gain of the signals. In some embodiments of the invention, ECG data is used in obtaining the measures, for example EGG data is used to calibrate the timing of a feature of the signal relative to the cardiac cycle.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

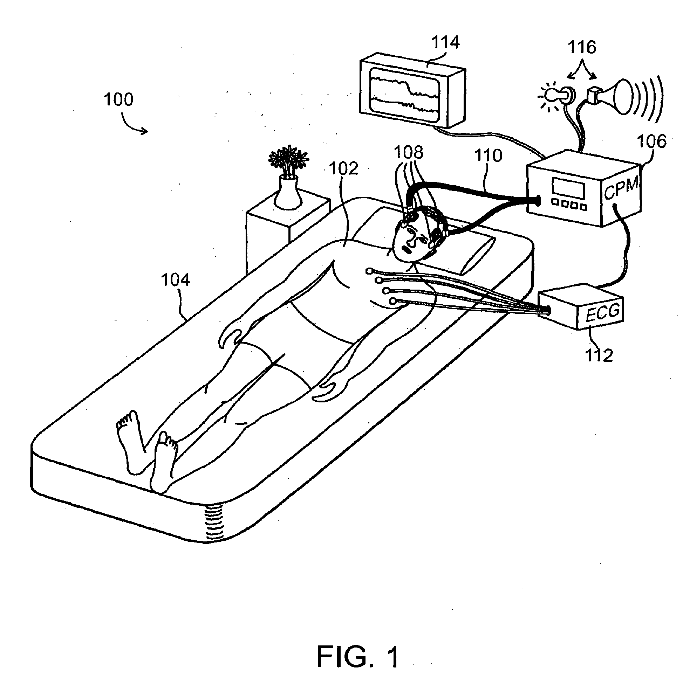

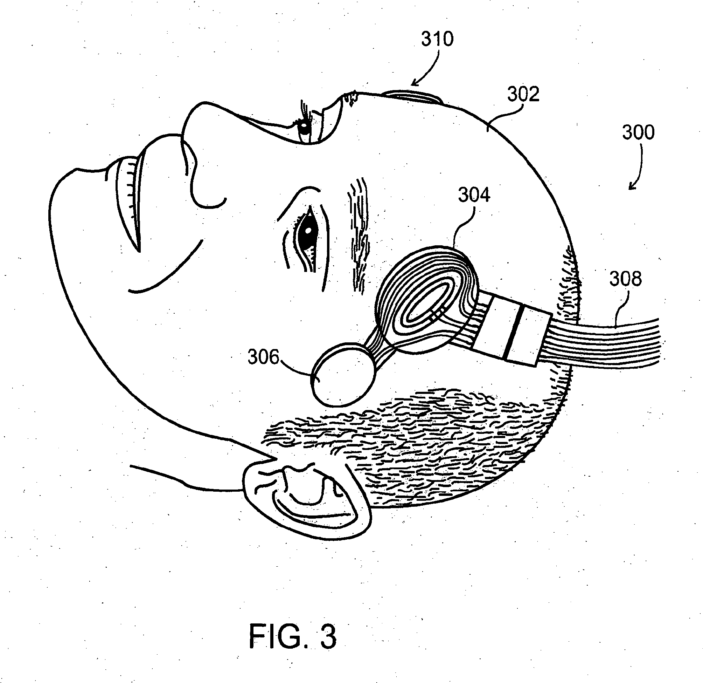

Referring now to the drawings, FIG. 1 illustrates a cerebral perfusion monitoring system 100, being used to monitor an acute stroke patient 102, optionally continuously, while lying on a bed 104, for example in a hospital. Patient 102 is, for example, an ischemic stroke patient, optionally a patient who has received thrombolytic therapy and is particularly prone to developing a cerebral hemorrhage. Alternatively, patient 102 is an SAH patient, who may be prone to vasospasm. A controller 106 is connected to sensors 108, placed on the patient's head, by cables 110. The controller is, for example, a general-purpose computer, or a specially dedicated circuit. The sensors include electrodes for IPG, and/or PPG sensors, which generate IPG and PPG signals analyzed by controller 106. An example of suitable sensors is shown in more detail below, in FIGS. 3-5. Optionally, an ECG device 112 is connected to ECG electrodes placed on the patient's chest, and ECG data is used by controller 106 in analyzing the signals from sensors 108. Optionally, a display screen 114 displays one or more cerebral hemodynamic parameters or other measures, as a function of time, calculated by controller 106 from the sensor data. Controller 106 activates one or more alert devices 116, for example a flashing light or an audible alarm, to alert medical personnel, if one or more measures of cerebral hemodynamics, calculated by controller 106, change by an amount and in a direction to indicate that the patient requires medical intervention, according to a rule programmed into controller 106. A person responding to the alert may be able to see at a glance the change in the patient's condition that caused the alert, by looking at display screen 114. Optionally, the alert device comprises an on-screen icon or similar element that appears, for example, on a display screen at a nurses' station. Optionally, the on-screen element also provides information about the values of the measures of cerebral hemodynamics that triggered the alert.

Although patient 102 is shown lying down in FIG. 1, system 100 may also be used to monitor patients who are ambulatory.

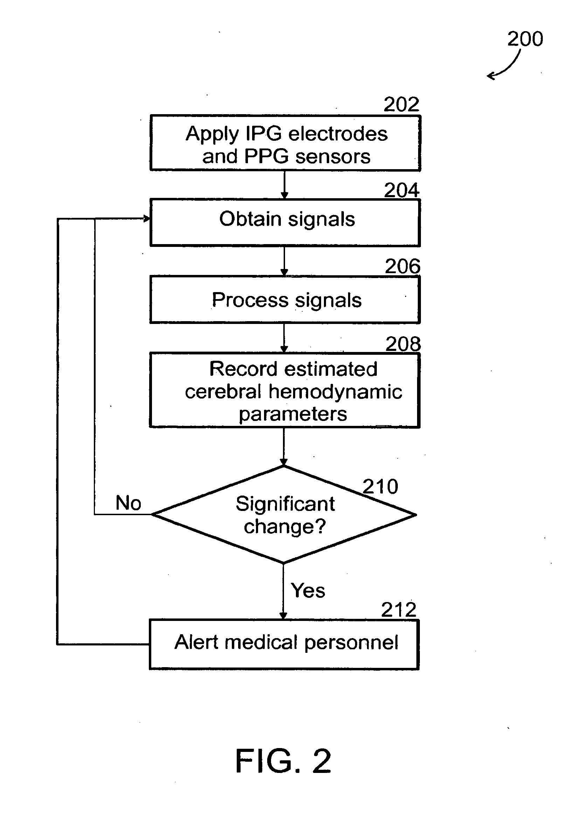

FIG. 2 shows a flowchart 200 of a method of monitoring a patient, used by system 100. At 202, IPG electrodes and/or PPG sensors are placed on the patient's head. In some embodiments of the invention, electrodes and/or PPG sensors may also be placed on the patient's neck, for example to measure a signal of blood flow in the carotid artery or another artery in the neck. The electrodes and sensors are optionally placed on the patient after admission to the hospital, and optionally after any initial tests are done, such as perfusion CT or MRI imaging, and after any initial treatment is administered, for example thrombolytic therapy. As described in the co-filed application "Diagnosis of Acute Strokes," a cerebral perfusion monitoring system similar to system 100 may also be used to evaluate stroke patients initially, before or upon admission to the hospital, and optionally electrodes and sensors already placed on the patient's head for that purpose are left in place when the patient is transferred to a hospital ward and monitored by system 100. Details of how electrodes and sensors are placed on the patient's head are described below in connection with FIG. 3.

At 204, one or more IPG signals from the IPG electrodes, and/or one or more PPG signals from the PPG sensors, are obtained by controller 106, and are processed by the controller at 206. Exemplary details of how this may be done are given below, under the heading "Exemplary methods of analyzing IPG and PPG signals." Controller 106 calculates from the signals one or more estimated cerebral hemodynamic parameters, such as CBF, CBV, MTT, and TTP, or other measures of cerebral hemodynamics, which can be used to detect medically significant changes in the patient's condition. The inventors have found, in clinical tests, that estimates of regional, hemispheric and global cerebral hemodynamic parameters, calculated from IPG and PPG signals, have correlations of about 0.5 to 0.7 with the same cerebral hemodynamic parameters measured by perfusion CT, across a sample of many different patients, with the parameters varying over a range of a factor of 2 or 3. It is likely that even higher correlations would be found between the estimates and the parameters for a given patient, if the parameters were to change over time.

The estimated cerebral hemodynamic parameters or other measures are optionally recorded at 208. This is done, for example, at regular frequent intervals, and the recorded data is used, for example, to create plots of the measures over time, for display screen 114.

At 210, the current estimates of the parameters or other measures are compared with past values, recorded at 208. If one or more of the measures have changed by too great an amount, either relatively or absolutely, then an alert is optionally given to medical personnel at 212, for example by sounding an alarm. The alert is optionally triggered automatically by controller 106, according to one or more criteria stored in a memory of controller 106, in the form of an algorithm, or a table, or a fuzzy logic condition, etc. The criteria may be personalized for different patients, depending for example on a diagnosis of their condition, or on clinical symptoms, and may be programmable by a physician.. For example, an alert may be given if an estimate of CBF, CBV, MTT, or TTP changes by more than 10% of its initial value, or by more than 20%, or by more than 30%, optionally only in a direction that indicates a worsening of the patient's condition, for example a drop in CBF or CBV, or an increase in MTT or TTP. Optionally, the alert is only given if the change persists for a minimum period, for example for at least 1 minute, or at least 5 minutes, or at least 10 minutes, or at least 20 minutes, or at least 30 minutes. Requiring such a waiting period may reduce false alarms, while still allowing a timely medical response when one is needed. An alert may also be given if a cerebral hemodynamic parameter fails to show an improvement in the patient's condition when it is expected to, for example if CBF fails to increase within one hour, or another period, after thrombolytic therapy is administered. An alert may also be given if a cerebral hemodynamic parameter jumps around in value more than usual, for example with at least twice its usual standard deviation, even without showing a trend in one direction, since this may indicate instability and incipient change in cerebral blood circulation.

For some parameters, a change in either direction may be a reason to give an alert. For example, a decrease in CBF may indicate an ischemic stroke, while an increase in CBF may indicate an increase in blood pressure that could increase the chance of a cerebral hemorrhage. Alternatively, an alert is given even for a change in a measure that indicates an improvement in the patient's condition. For example, if the patient received thrombolytic therapy, then an alert may be given if CBF increases to normal levels, indicating that the blocked artery recanalized due to the therapy. In response, perhaps after verifying the recanalization with other tests, the patient may be moved out of the Intensive Care Unit, or his treatment regimen may be changed to reflect the change in risks and tradeoffs.

Optionally, the threshold of change needed to trigger an alert is smaller if the change occurs over a shorter time. Optionally, an alert is given whenever a parameter goes beyond an absolute threshold, regardless of the amount change. Or, an alert is given whenever the parameter either goes beyond an absolute threshold or changes by a certain relative or absolute amount. For example, if an estimated value of regional CBF falls below a certain value, such as 20 milliters per 100 grams per minute, this may indicate a need for medical intervention, even if the change in regional CBF was not very great. The values of parameters or changes in parameters that trigger an alert optionally depend on the values of one or more other measures of cerebral hemodynamics, or on other medical parameters that are monitored, such as pulse rate, blood pressure, or body temperature.

FIG. 3 shows a combination sensor 300 for a cerebral perfusion monitor system, in place on the head of a patient 302. Another combination sensor 310, optionally a mirror image of sensor 300, is optionally used on the other side the patient's head, and is mostly hidden in FIG. 3. This sensor design is optionally used for sensors 108 in FIG. 1. Sensor 300 comprises an IPG electrode structure 304, optionally elliptical in shape, and optionally placed at a corner of the patient's forehead, optionally with an electrically conductive gel to assure good electrical contact with the skin. A PPG sensor 306, optionally circular, is optionally placed on the patient's temple. A cable 308 connects sensor 300 to the controller of the cerebral fusion monitor, for example controller 106 in FIG. 1. The cable optionally contains eight wires, including two wires used for electrode 304, and four wires used for PPG sensor 306 (two wires each for a light source and a light detector). Two of the wires in the cable are not used in sensor 300, but are included for use in a new design, under development, that will use two IPG electrodes on each side of the head.

Alternatively, any other design of IPG electrodes and/or PPG sensors, combined in one structure or separate, may be used, including any prior art design or off-the-shelf design for IPG electrodes and/or PPG sensors. The system need not use both IPG electrodes and PPG sensors, but optionally only uses one or the other.

The combination sensors used on the two sides of the patient's head are optionally placed at positions and orientations that are mirror images of each other, or nearly mirror images of each other, with respect to the bilateral symmetry plane of the head. Similarly, the two combination sensors are constructed to be mirror images of each other, or nearly mirror images of each other. Using sensors with such symmetry in design and location has the potential advantage that, by comparing measurements that are substantially mirror images of each other, they can be used to detect even small asymmetries in blood circulation in the head, which could be indicative of a stroke. In cases where the electrode and sensor configurations are said to be "nearly mirror images," the corresponding electrodes and sensors on the two sides of the head are all placed at locations that are mirror images of each other, to within 2 cm, or 1 cm, or 5 mm, or 2 mm, or 1 mm, or to within whatever precision the head is bilaterally symmetric. Alternatively, the corresponding electrodes and sensors are close enough to being placed in mirror image positions, that any differences in left and right hemisphere cerebral hemodynamic parameters inferred from the IPG and PPG signals from those misplaced sensors and electrodes will be small, by at least a factor of 2, or 5, or 10, or 20, compared to real differences in left and right hemisphere cerebral hemodynamic parameters typically found in ischemic stroke patients, or compared to the ranges in the values of these parameters typically seen among a random sample of ischemic stroke patients. Two measurements are "substantially mirror images of each other" if they are made with corresponding sensors and/or electrodes that are nearly mirror images in their configuration. Two measurements that are mirror images of each other, but are not identical, because each of the measurements is asymmetrical with respect to the bilateral symmetry of the head, should produce identical signals if the blood circulation in the head is bilaterally symmetric, as it normally is in a healthy subject. Any differences in such pairs of signals can reveal asymmetries in blood circulation in the head.

In some patients, previous trauma to the scalp or the brain, or previous brain surgery, may cause large asymmetries in the impedance of the head, so that symmetry in cerebral blood circulation cannot be inferred simply from differences in the impedance signals from two mirror image measurements. Similarly, massive and asymmetric scarring from a burn or other trauma may cause asymmetries in PPG signals from symmetrically placed sensors on opposite sides of the head. Even in these patients, it might be possible to detect changes in the asymmetry of cerebral blood circulation, from changes in a difference between mirror image IPG or PPG signals, if the initial differences are properly calibrated.

In some embodiments of the invention, additional electrodes and/or PPG sensors are used. For example, there may be two electrodes on each side of the head, allowing impedance measurements to be made symmetrically, for example locally on each side of the head. A number of such options are described in the cofled application titled "Measurement of Cerebral Hemodynamic Parameters," cited above. As used herein, an impedance measurement is called "asymmetric" if it is neither symmetric (such as current going from the middle of the forehead to the back of the head) or antisymmetric (such as current going from the right temple to the left temple).

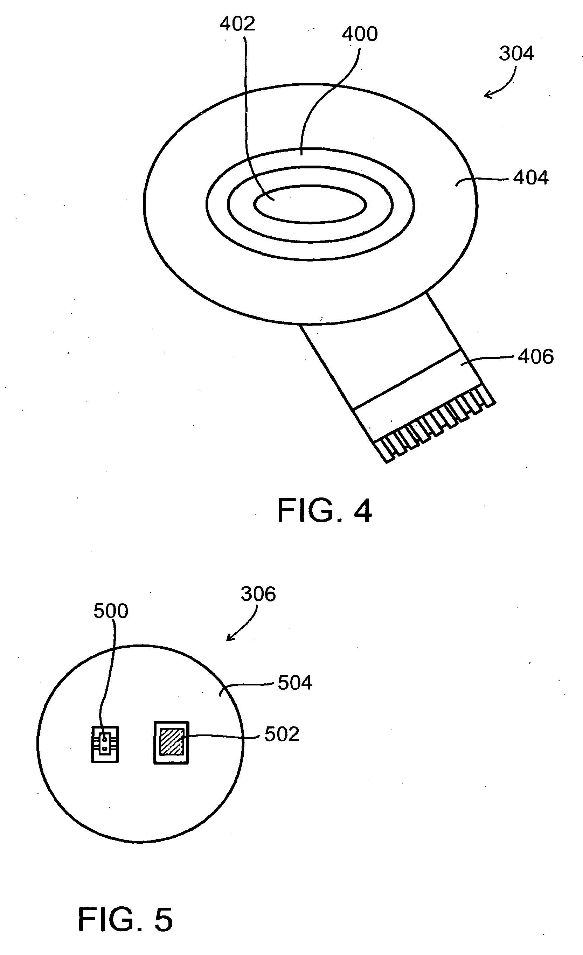

FIG. 4 shows electrode structure 304 in more detail. An elliptical ring-shaped current electrode 400 surrounds an elliptical voltage electrode 402. One of the wires in cable 308 connects to the current electrode, which passes current through the head, and one of the wires connects to the voltage electrode, which measures electric potential through a high impedance circuit, and passes very little current. Both are imbedded in an insulating holder 404, and a connector 406 snaps into a connector on the end of able 308, shown in FIG. 3. Some of the potential advantages of using a ring-shaped current electrode surrounding a central voltage electrode are described in two related patent applications cited above, US patent application 10/893,570, published as US2005/0054939, and PCT application PCT/IL2005/000632, published as WO2006/011128, although in those applications the electrodes are circular rather than elliptical. The ring-shaped current electrode may produce a broader distribution of current, resulting in more current going through the brain and less current going through the scalp, than if a more compact current electrode of the same area were used. The separate high-impedance voltage electrode, insulated from the current electrode, may effectively measure the voltage drop across the interior of the skull, with relatively little less contribution from the high impedance skin and skull, than if the same electrode were used for passing current and measuring voltage. For safety reasons, the electrodes use a frequency of at least a few kHz, and currents no greater than 2.5 mA. For the test data shown below in the Examples, a frequency of 25 kHz and current of 1 mA or less was used.

FIG. 5 shows a more detailed view PPG sensor 306, showing the surface of the sensor that is in contact with the skin. The sensor comprises a red LED 500, and a photodiode 502, imbedded in an opaque holder 504 that keeps out stray light. A suitable LED is, for example, model TF281-200, sold by III-V Compounds. A suitable photodiode is, for example, model TFMD5000R, also sold by III-V Compounds. Red light from the LED scatters from blood in the skin, with relatively little absorption compared to blue or green light. The amplitude of scattered light detected by the photodiode, which is optionally further shielded from stray light by a red filter that covers it, increases with increasing blood volume in the skin in the immediate vicinity of the LED and photodiode, and exhibits a characteristic rising and falling pattern over a cardiac cycle.

Conditions that can be detected by cerebral perfusion monitor

Among the conditions that system 100 could be used to detect, using the method of flowchart 200, are:

1) New ischemic stroke in an ischemic stroke patient. A new ischemic stroke can cause a sudden decrease in regional and hemispheric CBF, and a corresponding increase in regional and hemispheric TTP, both in the central core of the ischemia where tissue is likely to progress to an infarction, and in the penumbra where blood flow is reduced, but the tissue could recover if the blood clot can be removed. CBV, on the other hand, tends to be low only in the central core of the ischemia, but at near normal levels in the penumbra. A sudden decrease in CBF and increase in TTP, detected by system 100, could indicate a new ischemic stroke, and the magnitude of the decrease in CBV could indicate the relative size of the core and the penumbra. Once medical personnel have been alerted to this possibility in timely fashion, for example within an hour of the occurrence of the ischemia, or within 30 minutes, or 15 minutes, the nature of the event can be verified, and its precise location can be found, by techniques such as perfusion CT and MRI. Particularly if there is a large penumbra, prompt intervention can prevent further damage. For example, an endovascular procedure such as an embolectomy can be used to attempt to remove the blockage. The window of opportunity to remove the blockage, in order to prevent permanent damage to the penumbra, may be about 3 hours.

2) Vasospasm in a SAH patient. Vasospasm, a common complication of SAH, is expected to produce similar effects on cerebral hemodynamic parameters as an ischemic stroke, and its occurrence can be verified, and location found, using CT-Angio imaging, for example. Prompt medical intervention can save viable brain tissue. Possible treatment includes triple H therapy, vasodilator drugs, and endovascular angioplasty.

3) Hyperperfusion in an ischemic stroke patient. An increase in CBF, above normal values, for example by 10%, 20%, 30%, 50% or 100%, may indicate an increase in blood pressure, which can increase the risk of a hemorrhagic transformation of the ischemia, or of a new cerebral hemorrhage. If detected by system 100, and verified by other tests, it can be treated by blood pressure lowering medication.

4) Hemorrhagic transformation of ischemic stroke, or edema. It is expected that a cerebral hemorrhage may gradually decrease CBF, as intracranial pressure builds up, over an hour or several hours for example. Edema may have a similar effect. Any sufficiently large decrease in CBF could be detected by system 100, and once medical personnel are alerted, imaging techniques such as CT or MRI could be used to find the cause, and it can be treated in timely fashion, within an hour or a few hours.

5) Success or failure of thrombolytic therapy in first few hours. Thrombolytic therapy is normally administered intravenously. A patient receiving thrombolytic therapy can be monitored immediately afterward using system 100, to see if the blood clot has dissolved, as indicated by a recovery of CBF for example. If the blood clot fails to dissolve in an hour or two, thrombolytic therapy can be administered again, through a femoral artery, while continuing to monitor the patient by system 100. If the blood clot still fails to dissolve, an endovascular procedure can be used to try to remove the blood clot. This kind of monitoring is sometimes done for very high risk patients, using CT-Angio imaging instead of using system 100 to determine whether the blood clot has dissolved, but that is too expensive to do routinely for most patients. Monitoring using system 100 is much less expensive and can be done routinely for all patients receiving thrombolytic therapy.

Exemplary methods of analyzing IPG and PPG signals

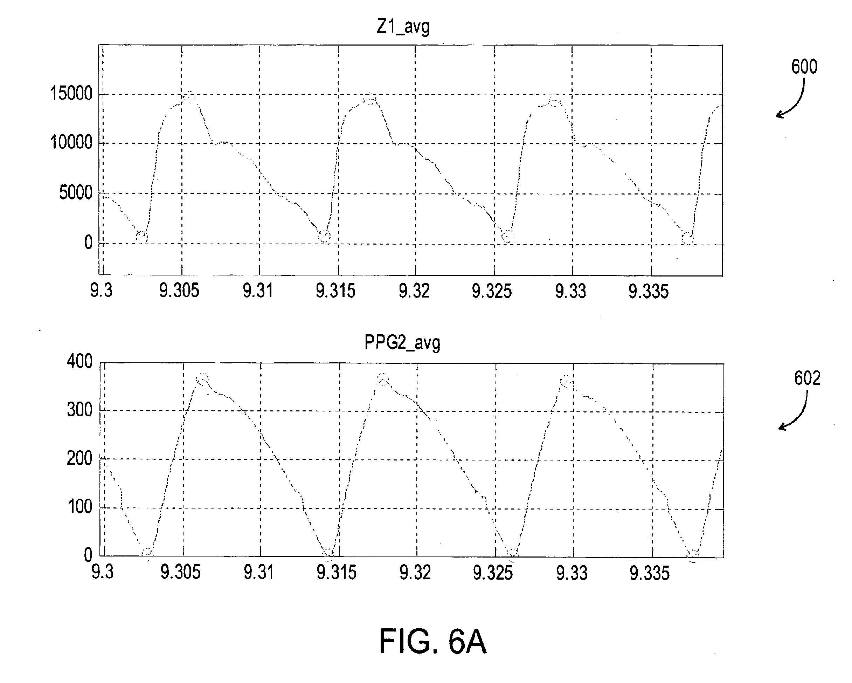

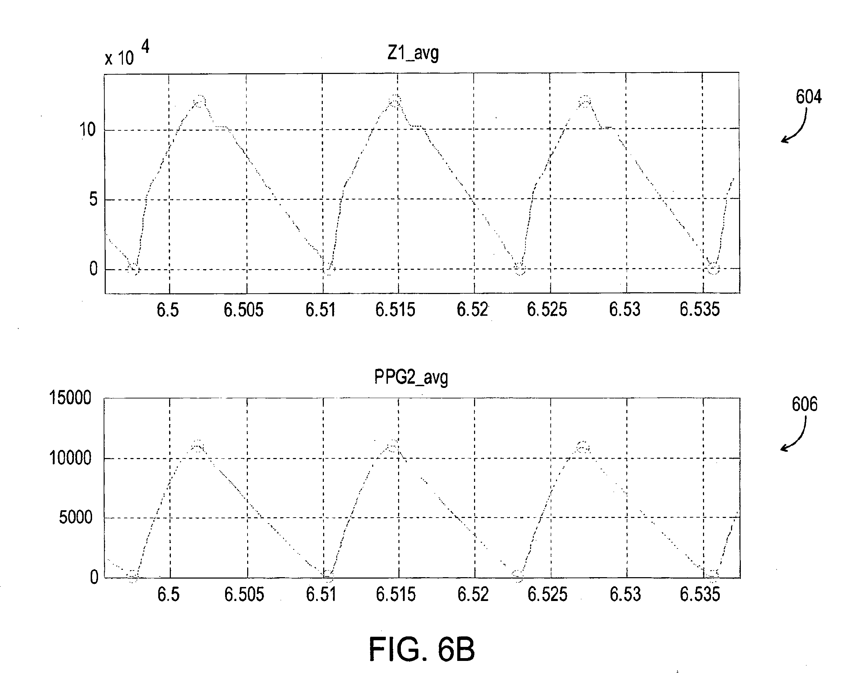

A number of methods of analyzing IPG and PPG signals have been found by the inventors to be useful for estimating standard cerebral hemodynamic parameters, as shown by results of a clinical study described below in the Examples. Most of these methods involve analysis of features of the signal that approximately repeat each cardiac cycle. For those features, noise can optionally be reduced by detrending the signal, so that it is always at the same level at the diastolic point of each cycle, by throwing out noisy or unusual cardiac cycles, and by taking a running average of the signals from multiple cardiac cycles in phase with each other, for example taking a running average over 9 cardiac cycles. As described in related PCT application PCT/IL2007/001421, cited above, published as WO2008/072223, the result is a relatively low noise signal as a function of cardiac phase, which rises over a relatively short rise time from its minimum value at the diastolic point to a maximum value at the systolic point, and then falls over a longer fall time back to its minimum value at the next diastolic point. Examples of such detrended and averaged IPG and IPG signals are shown below in FIGS. 6A and 6B. The signal used for the analysis need not be a linearly amplified signal coming directly from the IPG electrodes and PPG sensors, but may be nonlinearly distorted in any manner.

An effective robust rise time interval may be defined, which may further reduce the effect of noise on the signal analysis. For example, the robust rise time interval begins when the signal is a certain fraction of its total range (maximum minus minimum) above the minimum value, for example 5% or 10% or 15% or 20% above the minimum. The robust rise time interval optionally ends when the signal first reaches a point a certain fraction of its total range below the maximum, for example 5%, 10%, 15%, 20%, 25% or 30% below the maximum. For the data analyzed below in the Examples, the robust rise time interval is defined as extended from a point 10% above the minimum to a point 20% below the maximum.

Other effective rise times are defined as ending at the point of maximum slope, or at the first local peak. With these definitions of the end of the effective rise time, the rise time interval, and other quantities which depend on it, may be less subject to being changed by noise, than if the rise time were defined as ending at the global maximum of the signal.

Characteristics of the signal in an effective rise time interval may be compared to similar characteristics of the signal in an effective fall time interval, which may optionally be defined as any part of the cardiac cycle excluding the effective rise time interval. For example, a ratio of the effective rise time interval to the effective fall time interval may be calculated, or a ratio of the signal integrated over the effective rise time interval to a ratio of the signal over the effective fall time interval. Such ratios are respectively related in a simple way to the effective rise time normalized to the whole cardiac period, and to the signal integrated over the effective rise time, normalized to the signal integrated over the whole cardiac period. The latter measure has been found to be particularly useful for estimating some standard cerebral hemodynamic parameters, as is described below in the Examples.

Another measure used in the Examples is a normalized curvature of the signal during an effective rise time interval. The curvature is defined, for example, by first fitting the signal during the rise time interval to a straight line, then fitting the signal during the rise time interval to a parabola, and taking the difference in the cardiac phase, or time, where the two fits cross a level halfway between the minimum and maximum of, the signal. This difference may be normalized to the length of the rise time interval. This definition of curvature may be less sensitive to noise than simply taking the average second derivative of the signal during an effective rise time interval.

It may be useful to compare measures calculated by the same or substantially the same algorithm from two different signals, and this can serve as a measure based on both signals. (Two algorithms may be considered substantially the same if they yield similar results from a given signal, at least for most signals that are likely to occur.) For example, if the measure for each signal is an effective rise time defined in a particular way, then a measure based on two signals could be the ratio of the effective rise time for the first signal, to the effective rise time defined in the same way, or substantially the same way, for the second signal. Similarly, if the measure for each signal is the normalized signal integrated over the robust rise time described above, then the measure based on both signals could be the ratio of that normalized integral for the first signal, to the normalized integral from the second signal, defined in the same way, or substantially the same way. The two signals could be, for example, an IPG signal and a PPG signal measured on the same side of the head, or an IPG signal measured symmetrically across the head and a PPG signal measured on one side of the head, or two signals of the same modality measured on opposite sides of the head. If the measure only uses a signal measured on one side of the head, then the signal may be on the same side of the head as the suspected stroke, based on clinical data such as hemiplegia, or it may be on the opposite side of the head from the suspected stroke. It should be noted that blood circulation patterns on the side of the head opposite to a stroke are also generally affected by the stroke, because, for example, an ischemia on one side of the head may cause greater than normal blood flow on the other side of the head.

As used herein, a procedure is said to comprise comparing two signals when the procedure comprises calculating a difference between the two signals, or calculating a ratio of the two signals, or calculating any quantity that depends on how the two signals are different from each other.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions, illustrate some embodiments of the invention in a non limiting fashion.

A clinical study was performed, using stroke patients, in which certain standard cerebral hemodynamic parameters were measured by perfusion CT, and were also estimated using various dimensionless measures based on IPG and PPG signals as functions of cardiac cycle phase. The IPG electrodes and PPG sensors were configured as shown in FIG. 3, and the IPG signals were found using up to 1 mA of current, at about 25 kHz. The signals were detrended, setting their minimum for each cardiac cycle to the same level, and in some but not all cases several consecutive cardiac cycles were averaged together, in phase, to reduce noise while retaining the shape of the signal as a function of cardiac cycle phase. A best linear fit and correlation were calculated for the dimensionless measures based on the IPG and PPG signals, and the values of the parameters measured by perfusion CT. Correlations found ranged from approximately 0.5 to 0.7, with values of the parameters generally ranging over a factor of about 2 or 3, or occasionally more, among the different patients in the sample. The best linear fits listed here could be used as a starting point for providing estimates of these cerebral hemodynamic parameters from IPG and PPG data. For example, if the normalized integral over the robust rise time is found to be exactly the same for the IPG signal and the IPG signal on the same side of the head as the stroke, then measure #2, in the list below, would be equal to 1.0, and substituting this value for the measure into the best linear fit for measure #2, Measure = -Parameter/6.9 + 1.49, would imply that the value of the parameter, in this case global CBV, is probably about 3.5. Standard units are used for the parameters: milliliters per 100 grams of tissue for CBV, milliliters per 100 grams of tissue per minute for CBF, and seconds for TTP.

1) This measure was the ratio of a measure based on the IPG signal across the head, to a measure based on the PPG signal on the side of the head opposite to the stroke. For each of these signals, the measure was a rise time interval starting at the diastolic point, and ending at the point of maximum slope. This measure was used to estimate the parameter hemispheric CBV on the stroke side. The correlation was $R^2 = 0.54$, and the best linear fit was:

$$Measure = Parameter/4.8 + 0.06$$

with the hemispheric CBV ranging from about 2 to 4.5 milliliters per 100 grams, for the bulk of the patients in the sample.

2) This measure was the ratio of a measure based on the IPG signal across the head, to a measure based on the PPG signal on the same side of the head as the stroke. For each of these signals, the measure was the normalized integral of the signal over the robust rise time interval, defined above. This measure was used to estimate the parameter global CBV. The correlation was $R^2 = 0.72$, and the best linear fit was:

$$Measure = -Parameter/6.9 + 1.49$$

with the global CBV ranging from about 2 to 4.5 milliliters per 100 grams, for the bulk of the patients in the sample.

3) This measure was the ratio of a measure based on the IPG signal across the head, to a measure based on the IPG signal on the opposite side of the head from the stroke. For each of these signals, the measure was the normalized integral of the signal over the robust rise time interval, defined above. This measure was used to estimate the parameter global CBV. The correlation was $R^2 = 0.59$, and the best linear fit was:

$$Measure = -Parameter/8.3 + 1.4$$

4) This measure was the normalized integral of the signal over the robust rise time interval, defined above, for the PPG signal on the same side of the head as the stroke. This measure was used to estimate hemispheric CBF on the same side of the head as the stroke. The correlation was $R^2 = 0.56$, and the best linear fit was:

$$Measure = Parameter/650 + 0.12$$

with the hemispheric CBF ranging from 13 to 40 milliliters per 100 grams per minute, for the bulk of the patients in the sample.

5) This measure was the normalized integral of the signal over the robust rise time interval, defined above, for the PPG signal on the same side of the head as the stroke. This measure was used to estimate hemispheric TTP on the same side of the head as the stroke. The correlation was $R^2 = 0.56$, and the best linear fit was:

$$Measure = Parameter/420 + 0.08$$

with the hemispheric TTP ranging from 20 to 40 seconds, for the bulk of patients in the sample.

6) This measure was the normalized integral of the signal over the robust rise time interval, defined above, for the IPG signal across the head. This measure was used to estimate global TTP. The correlation was $R^2 = 0.46$, and the best linear fit was:

$$Measure = Parameter/280 + 0.04$$

with the global TTP ranging from 25 to 35 seconds, for the bulk of the patients in the sample.

7) This measure was the normalized rise time curvature of the signal, defined above, for the PPG signal on the same side of the head as the stroke. This measure was used to estimate the ratio of regional CBF on the same side of the head as the stroke, to global CBF, a quantity with a range of about a factor of 8 over the patients in the sample. The correlation was $R^2 = 0.53$, and the best linear fit was:

$$Measure = Parameter/21.6 + 0.017$$

with the ratio of regional to global CBF ranging from 0.1 to 0.8 for the bulk of the patients in the sample.

The relatively high correlations found between the measures of the IPG and IPG signals, and the cerebral hemodynamic parameters, show that it is already feasible to obtain useful estimates of cerebral hemodynamic parameters from IPG and IPG signals. In the near future, when more refined techniques for measuring IPG and IPG signals, and better measures derived from those signals, may be available, even more precise estimates of cerebral hemodynamic parameters may be possible.

FIGS. 6A and 6B show plots of IPG and PPG signals for two ischemic stroke patients who participated in the clinical study: FIG. 6A shows a plot 600 of the IPG signal measured across the head, and a plot 602 of the PPG signal measured on the same side of the head as the stroke, for a patient with unusually high global CBV, 5.3 milliliters per 100 grams of tissue, as measured by perfusion CT. The time is given in minutes, and the amplitudes of the signals are in arbitrary units. Noise has been reduced by taking a running average over 9 cardiac cycles, adding up the different cardiac cycles in phase. FIG. 6B shows a plot 604 of an IPG signal, and a plot 606 of a PPG signal, measured in the same way for a patient with unusually low global CBV, only 2.1 milliliters per 100 grams of tissue. The signals, especially the IPG signal, are visibly very different in the two patients, reflecting the large differences in their global CBV. The differences may be quantified by taking the normalized integral of the signal over a robust rise time, as described above. This quantity is 0.08 for the signal in plot 600, because the signal rises very quickly; 0.14 for the signal in plot 602; 0.21 for the signal in plot 604, which rises much more slowly than the signal in plot 600; an 0.19 for the signal in plot 606. The ratio of this quantity for the two signals provides a measure of 0.6 for the first patient, with global CBV parameter equal to 5.3, and a measure of 1.1 for the second patient, with global CBV parameter equal to 2.1. These quantities fit fairly well with the best linear fit, Measure = -Parameter/6.9 + 1.49, found for this parameter and measure in the clinical study, and one could have inferred the global CBV for these patients to fairly good approximation based on this relationship and on the IPG and PPG signals, even without making the much more expensive Perfusion CT measurement.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

To the extent that section headings are used, they should not be construed as necessarily limiting.

**Claims**

1. A system for monitoring an acute stroke patient, comprising:

    at least two IPG sensors (108) configured to pass current from a current source through a head of the patient to measure impedance;
    a controller (106) configured to:

        a)-obtain signals of impedance plethysmography (IPG) from the at least two PPG sensors (108) at least once an hour, for at least six hours;
        b)-process the IPG signals to obtain first and second measures of cerebral hemodynamics of the patient;

        c)-find a measure based on comparing the first and second measures; and
        d)-apply a rule about alerting or not alerting medical personnel based on any of values, amount of change, and direction and rate of change of the measure based on comparing the first and second measures,

        **characterized in that** the controller (106) is configured to apply a same or substantially same algorithm to the IPG signals to obtain the first and second measures, and the first and second measures each use an effective rise time of a cardiac cycle of the respective signal, or each use an integral of the respective signal over an effective rise time.

2. A system according to claim 1, wherein the controller (106) is configured to find the first and second measures and to apply the rule automatically without human intervention.

3. A system according to any of the preceding claims, wherein the patient is an ischemic stroke patient.

4. A system according to any of claims 1-2, wherein the patient is a subarachnoid hemorrhage (SAH) patient.

5. A system according to any of the preceding claims, wherein the measures comprise an estimate of one or more of global, hemispheric and regional measures of cerebral blood flow (CBF), of cerebral blood volume (CBV), of mean transit time (MTT), and of time to peak (TTP), and mathematical functions of the foregoing parameters singly or in any combination.

6. A system according to any of the preceding claims, wherein the signals comprise at least a first signal obtained from a measurement primarily of the left side of the head, and a second signal obtained from a measurement primarily on the right side of the head that is substantially a mirror image of the first measurement, and the controller is further configured to compare the first and second signals.

7. A system according to any of the preceding claims, wherein the signals comprise at least one signal obtained from an impedance measurement made substantially symmetrically or anti-symmetrically with respect to a bilateral symmetry plane of the patient's head.

8. A system according to any of the preceding claims, wherein the effective rise time interval begins when the signal first reaches a fixed percentage of the full range of the signal, above a minimum value of the signal.

9. A system according to any of the preceding claims, wherein the effective rise time interval ends when the signal first reaches a fixed percentage of the full range of the signal, below a maximum value of the signal.

10. A system according to any of the preceding claims, wherein the effective rise time interval ends at a maximum slope of the signal, or at a first inflection point of the signal with positive third derivative, or at as first local maximum of the signal, after the beginning of the effective rise time interval.

11. A system according to any of the preceding claims, wherein the controller (106) is further configured to find an integral of the signal over the effective rise time interval.

12. A system according to claim 11, wherein the controller (106) is further configured to compare the integral of said signal over the effective rise time interval to an integral of said signal over an effective fall time interval of a cardiac cycle, or over a whole cardiac cycle.

13. A system according to any of the preceding claims, wherein the controller (106) is further configured to find a curvature of the signal during the effective rise time interval.

14. A system according to any of the preceding claims, wherein the controller (106) is further configured to normalize a signal to obtain a measure that does not depend on a degree of amplification of the signal.

15. A system according to any of the preceding claims, wherein the controller (106) is further configured to normalize a time interval to a cardiac cycle period.

16. A system according to any of the preceding claims, wherein the controller (106) is further configured to receive an electrocardiogram (ECG) signal of the patient, and use the ECG signal to calibrate the timing of a feature of an IPG signal in a cardiac cycle.

17. A system according to any of the preceding claims, wherein the measures comprise an estimate of cerebral blood flow, and medical personnel are alerted when the estimate of cerebral blood flow falls by a predetermined relative amount that is at least 10% of an initial value of the estimate of cerebral blood flow.

18. A system according to claim 17, wherein the predetermined relative amount is not more than 30% of an initial value of the estimate of cerebral blood flow.

19. A system according to any of the preceding claims, wherein the measures comprise an estimate of cerebral blood flow, and medical personnel are alerted when the estimate of cerebral blood flow increases by a predetermined relative or absolute amount.

20. A system according to any of the preceding claims, wherein the signals comprise a signal obtained from a measurement made primarily of one side of the head, and the controller (106) is further configured to use at least said signal to find a measure that is an estimate of a hemispheric or regional cerebral hemodynamic parameter on the same side of the head, or on the opposite side of the head.

21. A system according to claim 20, wherein the hemispheric or regional cerebral hemodynamic parameter is on a side of the head in which clinical evidence indicates a stroke occurred.

22. A system according to any of the preceding claims, wherein. a first one of the signals is obtained from a measurement made substantially symmetrically on the head with respect to the bilateral symmetry plane, and a second one of the signals is obtained from a measurement made primarily on one side of the head.

23. A system according to any of the preceding claims, wherein the signals are both obtained from measurements made primarily on a same side of the head.

24. A system according to any of the preceding claims, wherein processing the one or more signals comprises finding an average second derivative of the signal during the effective rise time interval.

25. A system according to any of the preceding claims, further comprising said current source.

26. A method of monitoring an acute stroke patient, comprising:

a) obtaining signals of impedance plethysmography (IPG) from at least two IPG sensors (102) configured to pass current from a current source through a head of the patient, at least once an hour, for at least six hours;
b) applying a same or substantially same algorithm to the IPG signals to obtain first and second measures of cerebral hemodynamics of the patient;
c) finding a measure based on comparing the first and second measures; and
d) applying a rule about alerting or not alerting medical personnel based on any of values, amount of change, and direction and rate of change of the measure based on comparing the first and second measures;

wherein the first and second measures each use an effective rise time of a cardiac cycle of the respective signal, or each use an integral of the respective signal over an effective rise time.

**Patentansprüche**

1. System zur Überwachung eines Patienten mit akutem Schlaganfall, Folgendes umfassend:

mindestens zwei IPG-Sensoren (108), die dafür konfiguriert sind, Strom von einer Stromquelle durch das Herz eines Patienten zu leiten, um die Impedanz zu messen, einen Controller (106), der für Folgendes konfiguriert ist:

a) Erzielen von Signalen der Impedanzplethysmografie (IPG) von den mindestens zwei IPG-Sensoren (108) mindestens sechs Stunden lang mindestens einmal die Stunde,
b) Verarbeiten der IPG-Signale, um erste und zweite Messwerte von zerebralen Hämodynamiken des Patienten zu erzielen,
c) Finden eines Messwertes, der auf dem Vergleich des ersten und des zweiten Messwertes basiert, und
d) Anwenden einer Regel zur Alarmierung oder Nichtalarmierung des medizinischen Personals, basierend auf Werten und/oder Umfang der Veränderung und/oder Richtung und Geschwindigkeit der Veränderung des Messwertes, der auf dem Vergleich des ersten und des zweiten Messwertes basiert,

**dadurch gekennzeichnet, dass** der Controller (106) dafür konfiguriert ist, den gleichen oder im Wesentlichen gleichen Algorithmus auf die IPG-Signale anzuwenden, um den ersten und den zweiten Messwert zu erzielen, und dass der erste und der zweite Messwert jeweils eine effektive Anstiegszeit eines Herzzyklus des entsprechenden Signals oder jeweils ein Integral des entsprechenden Signals über eine effektive Anstiegszeit verwenden.

2. System nach Anspruch 1, wobei der Controller (106) dafür konfiguriert ist, den ersten und den zweiten Messwert zu finden und die Regel automatisch, ohne menschliches Eingreifen anzuwenden.

3. System nach einem der vorhergehenden Ansprüche, wobei der Patient ein ischämischer Schlaganfallpatient ist.

4. System nach einem der Ansprüche 1 bis 2, wobei der Patient ein Subarachnoidalblutungs-(SAB)-Patient ist.

5. System nach einem der vorhergehenden Ansprüche, wobei die Messwerte eine Schätzung eines oder mehrerer globaler, hemisphärischer oder lokaler Messwerte der zerebralen Durchblutung (CBF), des zerebralen Blutvolumens (CBV), der mittleren Durchgangszeit (MTT) und der Zeit bis zum Peak (TTP) und mathematischer Funktionen der genannten Parameter einzeln oder in einer beliebigen Kombination umfassen.

6. System nach einem der vorhergehenden Ansprüche, wobei die Signale mindestens ein erstes Signal umfassen, das aus einer Messung vorrangig der linken Seite des Kopfes erzielt wird, und ein zweites Signal, das aus einer Messung vorrangig der rechten Seite des Kopfes erzielt wird, die im Wesentlichen ein Spiegelbild der ersten Messung ist, und der Controller ferner dafür konfiguriert ist, das erste und das zweite Signal zu vergleichen.

7. System nach einem der vorhergehenden Ansprüche, wobei die Signale mindestens ein Signal umfassend, das aus einer Impedanzmessung erzielt wird, die in Bezug auf die beidseitige Symmetrieebene des Patientenkopfes im Wesentlichen symmetrisch oder gegensymmetrisch vorgenommen wird.

8. System nach einem der vorhergehenden Ansprüche, wobei die Zeitspanne des effektiven Anstiegs beginnt, wenn das Signal erstmalig einen feststehenden Prozentsatz des vollen Signalbereichs erreicht, der über einem Minimalwert des Signals liegt.

9. System nach einem der vorhergehenden Ansprüche, wobei die Zeitspanne des effektiven Anstiegs endet, wenn das Signal erstmalig einen feststehenden Prozentsatz des vollen Signalbereichs erreicht, der unter einem Maximalwert des Signals liegt.

10. System nach einem der vorhergehenden Ansprüche, wobei die Zeitspanne des effektiven Anstiegs bei einem maximalen Signalgefälle oder am ersten Wendepunkt des Signals mit dritter positiver Ableitung oder bei einem ersten örtlichen Maximum des Signals nach dem Beginn der Zeitspanne des effektiven Anstiegs endet.

11. System nach einem der vorhergehenden Ansprüche, wobei der Controller (106) ferner dafür konfiguriert ist, ein Integral des Signals über die Zeitspanne des effektiven Anstiegs zu finden.

12. System nach Anspruch 11, wobei der Controller (106) ferner dafür konfiguriert ist, das Integral des Signals über die Zeitspanne des effektiven Anstiegs mit einem Integral des Signals über eine Zeitspanne des effektiven Abfalls eines Herzzyklus oder über einen gesamten Herzzyklus zu vergleichen.

13. System nach einem der vorhergehenden Ansprüche, wobei der Controller (106) ferner dafür konfiguriert ist, eine Krümmung des Signals während der Zeitspanne des effektiven Anstiegs zu finden.

14. System nach einem der vorhergehenden Ansprüche, wobei der Controller (106) ferner dafür konfiguriert ist, ein Signal zu normalisieren, um einen Messwert zu erzielen, der nicht von einem Grad der Verstärkung des Signals abhängt.

15. System nach einem der vorhergehenden Ansprüche, wobei der Controller (106) ferner dafür konfiguriert ist, eine Zeitspanne zu einer Herzzyklusspanne zu normalisieren.

16. System nach einem der vorhergehenden Ansprüche, wobei der Controller (106) ferner dafür konfiguriert ist, ein Elektrokardiogramm-(EKG)-Signal des Patienten zu empfangen und das EKG-Signal zu verwenden, um den Zeitpunkt eines Merkmals eines IPG in einem Herzzyklus zu kalibrieren.

17. System nach einem der vorhergehenden Ansprüche, wobei die Messwerte eine Schätzung der zerebralen Durchblutung umfassen und medizinisches Personal alarmiert wird, wenn die Schätzung der zerebralen Durchblutung um ein festgelegtes relatives Maß fällt, das mindestens 10 % eines Anfangswertes der Schätzung der zerebralen Durchblutung ausmacht.

18. System nach Anspruch 17, wobei das festgelegte relative Maß nicht mehr als 30 % eines Anfangswertes der Schätzung der zerebralen Durchblutung beträgt.

19. System nach einem der vorhergehenden Ansprüche, wobei die Messwerte eine Schätzung der zerebralen Durchblutung umfassen und medizinisches Personal alarmiert wird, wenn die Schätzung der zerebralen Durchblutung um ein festgelegtes relatives oder absolutes Maß zunimmt.

20. System nach einem der vorhergehenden Ansprüche, wobei die Signale ein Signal umfassen, das aus einer Messung erzielt wird, die vorrangig an einer Seite des Kopfes vorgenommen wird, und der Controller (106) ferner dafür konfiguriert ist, mindestens das Signal zu verwenden, um einen Messwert zu finden, der eine Schätzung eines hemisphärischen oder regionalen zerebralen hämodynamischen Parameters an derselben Seite des Kopfes oder an der gegenüberliegenden Seite des Kopfes ist.

21. System nach Anspruch 20, wobei der hemisphärische oder regionale zerebrale hämodynamische Parameter an einer Seite des Kopfes liegt, in der ein klinischer Nachweis einen aufgetretenen Schlaganfall anzeigt.

22. System nach einem der vorhergehenden Ansprüche, wobei ein erstes der Signale aus einer Messung erzielt wird, die am Kopf im Verhältnis zur beidseitigen Symmetrieebene im Wesentlichen symmetrisch vorgenommen wird, und ein zweites der Signale aus einer Messung erzielt wird, die vorrangig an einer Seite des Kopfes vorgenommen wird.

23. System nach einem der vorhergehenden Ansprüche, wobei beide Signale aus Messungen erzielt werden, die vorrangig an derselben Seite des Kopfes vorgenommen werden.

24. System nach einem der vorhergehenden Ansprüche, wobei das Verarbeiten des einen oder der mehreren Signale das Finden einer durchschnittlichen zweiten Ableitung des Signals während einer Zeitspanne des effektiven Anstiegs umfasst.

25. System nach einem der vorhergehenden Ansprüche, ferner die Stromquelle umfassend.

26. Verfahren zum Überwachen eines Patienten mit akutem Schlaganfall, Folgendes umfassend:

a) Erzielen von Signalen der Impedanzplethysmografie (IPG) von mindestens zwei IPG-Sensoren (108), die dafür konfiguriert sind, Strom von einer Stromquelle durch das Herz eines Patienten zu leiten, mindestens sechs Stunden lang mindestens einmal die Stunde,
b) Anwenden eines gleichen oder im Wesentlichen gleichen Algorithmus auf die IPG-Signale, um einen ersten und einen zweiten Messwert zerebraler Hämodynamiken des Patienten zu erzielen,
c) Finden eines Messwertes, der auf dem Vergleich des ersten und des zweiten Messwertes basiert, und
d) Anwenden einer Regel zur Alarmierung oder Nichtalarmierung des medizinischen Personals, basierend auf Werten und/oder Umfang der Veränderung und/oder Richtung und Geschwindigkeit der Veränderung des Messwertes, der auf dem Vergleich des ersten und des zweiten Messwertes basiert,

wobei der erste und der zweite Messwert jeweils eine effektive Anstiegszeit eines Herzzyklus des entsprechenden Signals oder jeweils ein Integral des entsprechenden Signals über eine effektive Anstiegszeit verwenden.

## Revendications

1. Système de surveillance d'un patient subissant des crises aiguës, comprenant :

    au moins deux capteurs PGI (108) conçus pour faire passer du courant d'une source de courant à travers une tête du patient pour mesurer l'impédance ;
    un contrôleur (106) conçu pour :

    a) obtenir des signaux de pléthysmographie d'impédance (PGI) à partir des au moins deux capteurs PGI (108) au moins une fois par heure, pendant au moins six heures ;
    b) traiter les signaux PGI afin d'obtenir une première et une deuxième mesure de l'hémodynamique, cérébrale du patient ;
    c) trouver une mesure sur la base de la comparaison de la première et de la deuxième mesure ; et
    d) appliquer une règle concernant l'avertissement et le non-avertissement du personnel médical sur la base d'une valeur, d'une quantité de changement et d'une direction et vitesse de changement de la mesure sur la base de la comparaison de la première et de la deuxième mesure,

    **caractérisé en ce que** le contrôleur (106) est conçu pour appliquer un algorithme identique ou globalement identique aux signaux PGI afin d'obtenir la première et la deuxième mesure et la première et la deuxième mesure utilisent chacune un temps d'augmentation effective d'un cycle cardiaque du signal respectif, ou chacune utilisent une intégrale du signal respectif sur un temps d'augmentation effective.

2. Système selon la revendication 1, dans lequel le contrôleur (106) est conçu pour trouver la première et la deuxième mesure et pour appliquer la règle automatiquement sans intervention humaine.

3. Système selon l'une des revendications précédentes, dans lequel le patient est un patient atteint de crises ischémiques.

4. Système selon l'une des revendications 1 à 2, dans lequel le patient est patient atteint d'hémorragie subarachnoïde (HSA).

5. Système selon l'une des revendications précédentes, dans lequel les mesures comprennent une estimation d'une ou plusieurs mesures globales, hémisphériques et régionales du flux sanguin cérébral (CBF), un volume sanguin cérébral (CBV), un temps de transit moyen (MTT) et un temps de crête (TTP) et des fonctions mathématiques des paramètres précédents simplement ou dans une combinaison quelconque.

6. Système selon l'une des revendications précédentes, dans lequel les signaux comprennent au moins un premier signal obtenu à partir d'une mesure principalement du côté gauche de la tête et un deuxième signal obtenu d'une mesure principalement sur le côté droit de la tête qui est globalement une image en miroir de la première mesure et le contrôleur est en outre conçu pour comparer le premier et le deuxième signal.

7. Système selon l'une des revendications précédentes, dans lequel les signaux comprennent au moins un signal obtenu à partir d'une mesure d'impédance faite de manière globalement symétrique ou antisymétrique par rapport à un plan de symétrie bilatéral de la tête du patient.

8. Système selon l'une des revendications précédentes, dans lequel l'intervalle de temps d'augmentation effective commence lorsque le signal atteint d'abord un pourcentage fixe de la plage entière du signal, au-dessus d'une valeur minimum du signal.

9. Système selon l'une des revendications précédentes, dans lequel l'intervalle de temps d'augmentation effective se termine lorsque le signal atteint d'abord un pourcentage fixe de la plage entière du signal, en dessous d'une valeur maximum du signal.

10. Système selon l'une des revendications précédentes, dans lequel l'intervalle de temps d'augmentation effective se termine au niveau d'une pente maximum du signal ou au niveau d'un premier point d'inflexion du signal avec une troisième dérivée positive ou au niveau d'un premier maximum local du signal, après le début de l'intervalle de temps d'augmentation effective.

11. Système selon l'une des revendications précédentes, dans lequel le contrôleur (106) est en outre conçu pour trouver une intégrale du signal sur l'intervalle de temps d'augmentation effective.

12. Système selon la revendication 11, dans lequel le contrôleur (106) est en outre conçu pour comparer l'intégrale dudit signal sur l'intervalle de temps d'augmentation effective avec une intégrale dudit signal sur un intervalle de temps de diminution effective d'un cycle cardiaque ou sur un cycle cardiaque complet.

**13.** Système selon l'une des revendications précédentes, dans lequel le contrôleur (106) est en outre conçu pour trouver une courbure du signal pendant l'intervalle de temps d'augmentation effective.

**14.** Système selon l'une des revendications précédentes, dans lequel le contrôleur (106) est en outre conçu pour normaliser un signal afin d'obtenir une mesure qui ne dépend pas d'un degré d'amplification du signal.

**15.** Système selon l'une des revendications précédentes, dans lequel le contrôleur (106) est en outre conçu pour normaliser un intervalle de temps sur une période de cycle cardiaque.

**16.** Système selon l'une des revendications précédentes, dans lequel le contrôleur (106) est en outre conçu pour recevoir un signal d'électrocardiogramme (ECG) du patient et pour utiliser le signal ECG pour calibrer la synchronisation d'une caractéristique d'un signal PGI dans un cycle cardiaque.

**17.** Système selon l'une des revendications précédentes, dans lequel les mesures comprennent une estimation du flux sanguin cérébral et le personnel médical est alerté lorsque l'estimation du flux sanguin cérébral diminue d'une quantité relative prédéterminée qui représente au moins 10% d'une valeur initiale de l'estimation du flux sanguin cérébral.

**18.** Système selon la revendication 17, dans lequel la quantité relative prédéterminée n'est pas supérieure à 30% d'une valeur initiale de l'estimation du flux sanguin cérébral.

**19.** Système selon l'une des revendications précédentes, dans lequel les mesures comprennent une estimation du flux sanguin cérébral et le personnel médical est alerté lorsque l'estimation du flux sanguin cérébral augmente d'une quantité relative ou absolue prédéterminée.

**20.** Système selon l'une des revendications précédentes, dans lequel les signaux comprennent un signal obtenu à partir d'une mesure faite principalement d'un côté de la tête et le contrôleur (106) est en outre conçu pour utiliser au moins ledit signal pour trouver une mesure qui est une estimation d'un paramètre hémodynamique cérébral hémisphérique ou régional sur le même côté de la tête ou sur le côté opposé de la tête.

**21.** Système selon la revendication 20, dans lequel le paramètre hémodynamique cérébral hémisphérique ou régional est sur un côté de la tête sur lequel des preuves cliniques indiquent qu'une crise s'est produite.

**22.** Système selon l'une des revendications précédentes, dans lequel un premier signal est obtenu à partir d'une mesure faite de manière globalement symétrique sur la tête par rapport au plan de symétrique bilatéral et un deuxième signal est obtenu à partir d'une mesure faite principalement sur un côté de la tête.

**23.** Système selon l'une des revendications précédentes, dans lequel les signaux sont tous les deux obtenus à partir de mesures faites principalement sur le même côté de la tête.

**24.** Système selon l'une des revendications précédentes, dans lequel le traitement d'un ou plusieurs signaux comprend la recherche d'une deuxième dérivée moyenne du signal pendant l'intervalle de temps d'augmentation effective.

**25.** Système selon l'une des revendications précédentes, comprenant en outre ladite source de courant.

**26.** Procédé de surveillance d'un patient atteint de crises aiguë, comprenant :

a) obtention de signaux de pléthysmographie d'impédance (PGI) à partir d'au moins deux capteurs PGI (102) conçus pour faire passer du courant d'une source de courant à travers une tête du patient pour mesurer l'impédance, au moins une fois par heure, pendant au moins six heures ;
b) application d'un algorithme identique ou globalement identique aux signaux PGI afin d'obtenir une première et une deuxième mesure de l'hémodynamique cérébral du patient ;
c) recherche d'une mesure sur la base de la comparaison de la première et de la deuxième mesure ; et
d) application d'une règle concernant l'avertissement et le non-avertissement du personnel médical sur la base d'une valeur, d'une quantité de changement et d'une direction et d'une vitesse de changement de la mesure sur la base de la comparaison de la première et de la deuxième mesure ;

dans lequel la première et la deuxième mesure utilisent chacune un temps d'augmentation effective d'un cycle cardiaque du signal respectif ou utilisent chacune une intégrale du signal respectif sur un temps d'augmentation effective.

FIG. 1

200

202

Apply IPG electrodes
and PPG sensors

204

Obtain signals

206

Process signals

208

Record estimated
cerebral hemodynamic
parameters

210

Significant
change?

No

Yes

212

Alert medical personnel

FIG. 2

FIG. 3

EP 2 344 034 B1

402    400    304

404

406

# FIG. 4

306

500

504

502

# FIG. 5

Z1_avg

600

PPG2_avg

602

FIG. 6A

FIG. 6B

EP 2 344 034 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11610553 B
- US 20070287899 A
- WO 2008072223 A
- WO 02071923 A, Bridger
- WO 02087410 A, Naisberg
- WO 2006011128 A
- WO 2006134501 A
- US 2008200787 A1
- US 2008004904 A

- RU 2141249
- US 2007287899 A
- DE 10061189 A1
- US 61103287 B
- US 10893570 B
- US 20050054939 A
- IL 2005000632 W
- IL 2007001421 W

**Non-patent literature cited in the description**

- **CHRISTIAN BAUMGARTNER et al.** Functional Cluster Analysis of CT Perfusion Maps: A New Tool for Diagnosis of Acute Strokes. *J. of Digital Imaging,* 2005, vol. 18, 219-226
- **ROLAND BRUENING ; AXEL KUETTNER ; THOMAS FLOHR.** Protocols for Multislice CT. Springer, 2005, 96
- **ELLEN G. HOEFFNER et al.** Cerebral Perfusion CT: Technique and Clinical Applications. *Radiology,* 2004, vol. 231, 632-644
- **HIROSHI HAGIWARA et al.** Predicting the Fate of Acute Ischemic Lesions Using Perfusion Computed Tomography. *J. Comput. Assist. Tomogr,* 2008, vol. 32, 645-650
- **A. M. WEINDLING ; N. MURDOCH ; P. ROLFE.** Effect of electrode size on the contributions of intracranial and extracranial blood flow to the cerebral electrical impedance plethysmogram. *Med. & Biol. Eng. & Comput.,* 1982, vol. 20, 545-549
- **J. GRONLUND ; J. JALONEN ; I. VALIMAKI.** Transcephalic electrical impedance provides a means for quantifying pulsatile cerebral blood volume changes following head-up tilt. *Early Human Development,* 1997, vol. 47, 11-18
- High Frequency Variability of Transcephalic Electrical Impedance: A New Parameter for Monitoring of Neonatal Cerebral Circulation?. **J. GRONLUND et al.** Proceedings of the Annual International Conference of the Engineering in Medicine and Biology Society, Paris. IEEE, 29 October 1992, vol. 6, 2513-2515
- **W. TRACZEWSKI et al.** The Role of Computerized Rheoencephalography in the Assessment of Normal Pressure Hydrocephalus. *J. Neurotrauma,* 2005, vol. 22, 836-843

- **G. BONMASSAR ; S. IWAKI.** The Shape of Electrical Impedance Spectrosopy (EIS) is altered in Stroke Patients. *Proceedings of the 26th Annual Conference of IEEE EMBS, San Francisco, CA, USA,* 01 September 2004
- **YU. E. MOSKALENKO et al.** Slow Rhythmic Oscillations within the Human Cranium: Phenomenology, Origin, and Informational Significance. *Human Physiology,* 2001, vol. 27, 171-178
- **A. RAGAUSKAS et al.** Implementation of non-invasive brain physiological monitoring concepts. *Medical Engineering and Physics,* 2003, vol. 25, 667-687
- **KIDWELL CS et al.** Comparison of MRI and CT for detection of acute intracerebral hemorrhage. *JAMA,* 2004, vol. 292, 1823-1830
- **HOROWITZ SH et al.** Computed tomographic-angiographic findings within the first 5 hours of cerebral infarction. *Stroke,* 1991, vol. 22, 1245-1253
- The ATLANTIS, ECASS, and NINDS rt-PA study group investigators, Association of outcome with early stroke treatment: Pooled analysis of ATLANTIS, ECASS, and NINDS rt-PA stroke trials. *Lancet,* vol. 363, 768-774
- **ALBERS G et al.** Antithrombotic and thrombolytic therapy for ischemic stroke: The seventh ACCP conference on antithrombotic and thrombolytic therapy. *Chest,* 2004, vol. 126, 483-512
- **KOHRMANN M.** MRI versus CT-based thrombolysis treatment within and beyond the 3 hour time window after stroke onset: a cohort study. *Lancet Neurol,* 2006, vol. 5, 661-667

- **ALBERS GW et al.** Magnetic resonance imaging profiles predict clinical response to early reperfusion: The diffusion and perfusion imaging evaluation for understanding stroke evolution (DEFUSE) study. *Ann Neurol,* 2006, vol. 60, 508-517
- **JOHNSTON SC et al.** National stroke association guidelines for the management of transient ischemic attacks. *Ann Neurol,* 2006, vol. 60, 301-313

- **LOVETT DOUST J W et al.** Aspects of the cerebral circulation during non REM sleep in healthy controls and psychiatric patients, as shown by rheoencephalography. *PSYCHOPHYSIOLOGY,* 1975, vol. 12 (5), ISSN 0048-5772, 493-498